# EUROPÄISCHE PATENTSCHRIFT

(11) **EP 0 930 884 B1**
(45) Veröffentlichungstag und Bekanntmachung des Hinweises auf die Patenterteilung: **18.12.2002**
(21) Anmeldenummer: 97918877.8
(22) Anmeldetag: 02.10.1997
(51) Int. Cl.: A61K 31/55, A61P 25/00

(54) **CHRONISCHE VERWENDUNG VON LORAZEPAM UND DIPHENHYDRAMIN**
CHRONIC USE OF LORAZEPAM AND DIPHENHYDRAMINE
UTILISATION CHRONIQUE DU LORAZEPAM ET DE LA DIPHENEHYDRAMINE

(30) Priorität: 11.10.1996 CH 248096
(43) Veröffentlichungstag der Anmeldung: 28.07.1999
(73) Patentinhaber: MEDICHEMIE AG, CH-4107 Ettingen/Basel (CH)
(72) Erfinder: WIDAUER, Josef, Olaf, CH-4123 Allschwil (CH)
(74) Vertreter: Lauer, Joachim
(86) Internationale Anmeldenummer: CH9700368
(87) Internationale Veröffentlichungsnummer: WO98016231

(56) Entgegenhaltungen:
- EP-A- 0 151 245
- SALETU B ET AL: "COMPARATIVE DOUBLE-BLIND PLACEBO-CONTROLLED SLEEP LABORATORY STUDIES OF A COMBINATION OF LORAZEPAM AND DIPHENHYDRAMINE SM-1014 AND ITS SINGLE COMPONENTS" CURR THER RES CLIN EXP, 42 (6). 1987. 1037-1058., XP000675743
- GRUENBERGER J ET AL: "PHARMACODYNAMIC STUDIES OF A COMBINATION OF LORAZEPAM AND DIPHENHYDRAMINE AND ITS SINGLE COMPONENTS PSYCHOMETRIC AND PSYCHOPHYSIOLOGICAL DATA" CURR THER RES CLIN EXP, 44 (6). 1988. 938-965., XP000675741
- SALETU B ET AL: "PHARMACODYNAMIC STUDIES OF A COMBINATION OF LORAZEPAM AND DIPHENHYDRAMINE AND ITS SINGLE COMPONENTS ELECTROENCEPHALOGRAPHIC BRAIN MAPPING AND SAFETY EVALUATION" CURR THER RES CLIN EXP, 44 (6). 1988. 909-937., XP000675742

## Beschreibung

### TECHNISCHES GEBIET

Die Erfindung bezieht sich auf das Gebiet der Schlafmittel und betrifft insbesondere eine Mischung aus Lorazepam und Diphenhydramin oder einem Säureadditionssalz von Diphenhydramin zur Herstellung eines oral oder enteral verabreichbaren Schlafmittels.

### STAND DER TECHNIK

Aus der EP 0 151 245 B1 ist ein derartiges Schlafmittel bekannt, welches Lorazepam und Diphenhydramin oder ein Säureadditionssalz von Diphenhydramin im Gewichtsverhältnis 1:10 bis 1:75 enthält. Speziell für ein Schlafmittel, welches die genannten Bestandteile im Gewichtsverhältnis 1:25 enthielt, konnte in Experimenten gezeigt werden, dass seine jeweils einzeln für sich schlaffördernden Bestandteile Lorazepam und Diphenhydramin in der genannten Mischung in niedrigen Dosen eine überadditive schlaffördernde Wirkung haben, sich zu höheren hohen Dosen hin ihre akuten Toxizitäten dagegen progredient antagonisieren. Beim Menschen verursacht Lorazepam allein bereits in relativ schwacher Dosierung von 2 - 3 mg pro Einzeldosis zum Teil ausgeprägte direkte Nebenwirkungen wie Muskelrelaxationen, Ataxie sowie sedative Nachwirkungen (Hangover) "am Morgen danach". In akuten Tests an Versuchspersonen bzw. Patienten, welche unter deutlichen Ein- und Durchschlafstörungen verschiedener Ursache und Stärke litten, konnte für das Kombinationspräparat aus 1 mg Lorazepam und 25 mg Diphenhydramin die Wirkungssteigerung gegenüber reinem Lorazepam ebenfalls nachgewiesen werden, wobei die genannten Neben- bzw. Überhangwirkungen jedoch in wesentlich geringerem Masse auftraten. Die tierxperimentell festgestellte, sich offenbar antagonisierende Toxizität der beiden Komponenten bei hohen Dosen kann im Hinblick auf einen Missbrauch zu Suizidzwecken von Vorteil sein.

Als generell problematisch wird bei Schlafmitteln ihre Einnahme über einen längeren Zeitraum angesehen. Wohl wegen der Aufwendigkeit von Langzeittests liegen jedoch nur wenig gesicherte Erkenntnisse über die Langzeitwirkungen von Schlafmitteln vor. Die meisten Tests sind Akutstudien. Die verfügbaren Daten deuten generell darauf hin, dass grundsätzlich zumindest mit einer Beeinträchtigung der cognitiven Leistungsfähigkeit gerechnet werden muss. So konnte z.B. für Lorazepam in einer Dosierung von 1 - 2 mg bereits nach 7 Tagen eine signifikant schlechtere Hirnleistung, eine signifikant verlangsamte totale Reaktionszeit, eine beeinträchtigte motorische Reaktionszeit sowie eine Beeinträchtigung des Aufwachens festgestellt werden (Hindmarch et al., Neuropsychobiology 24, 84-89, (1990-1991).

Eine Beeinträchtigung der cognitiven Leistungsfähigkeit ist insbesonders bei älteren Menschen, bei denen diese Leistungsfähigkeit bereits alterbedingt nachgelassen hat, besonders relevant. In dieser Personengruppe treten andererseits chronische Schlafstörungen verstärkt auf. Von daher besteht das Bedürfnis, ein wirksames Schlafmittel zur Verfügung zu stellen, welches möglichst geringe Neben- oder Überhangwirkungen hat und vor allem auch bei längerfristiger Einnahme über mehrere Tage oder Wochen die cognitive Leistungsfähigkeit nicht negativ beinträchtigt.

### DARSTELLUNG DER ERFINDUNG

An der neurophysiologischen Abteilung der psychiatrischen Universitätsklinik Wien wurden kürzlich Versuche durchgeführt mit dem Ziel, die im Akutversuch nachgewiesene Wirkungssteigerung der Kombination aus Lorazepam und Diphenhydramin gegenüber reinem Lorazepam auch im Langzeitversuch an Patienten zu verifizieren, welche unter Schlafstörungen leiden. Die Versuche ersteckten sich über jeweils 6 Wochen, wobei das Kombinationspräparat in der Zusammensetzung von 1 mg Lorazepam und 25 mg Diphenhydramin pro Einzeldosis einer Patientengruppe A über vier Wochen verabreicht wurde. Einer Vergleichsgruppe B wurde parallel dazu reines Lorazepam in der Dosierung von 1 mg pro Einzeldosis verabreicht. Jeweils vor und nach der vierwöchigen Behandlungsperiode wurde den Patienten ein Placebopräparat verabreicht.

Neben anderen Parametern wurden diese Patienten über die gesamte Studiendauer einer Reihe wissenschaftlich anerkannter noopsychischer Tests unterzogen, um Aufschluss über die cognitiven Fähigkeiten tagsüber während der vierwöchigen Einnahme der Prüfpräparate zu erhalten. Es handelte sich um folgende psychometrischen Untersuchungen:
- GVG-AM
   allgemeine Merkfähigkeit
- GVG-ASS
   assoziierte Merkfähigkeit
- GVG-ZG
   Zahlengedächtnis
- Benton R. / Benton Test
   visuelles Gedächtnis, richtige Resultate
- Benton F. / Benton Test
   visuelles Gedächtnis, falsche Resultate

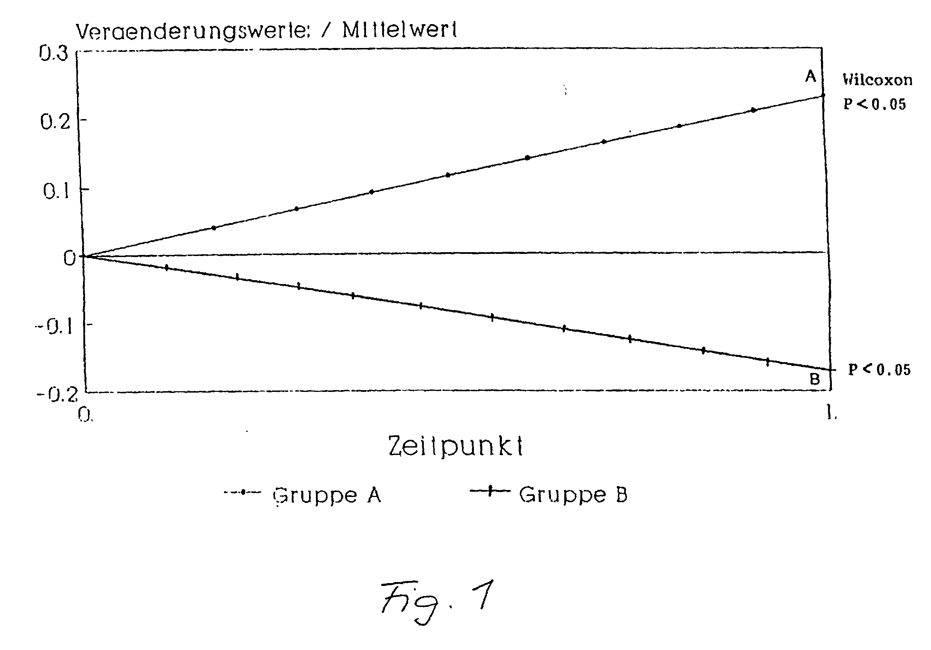

Die grafische Darstellung von Fig. 1 zeigt das Gesamtergebnis aller dieser Tests in relativen Einheiten entlang der Ordinate aufgetragen. Das dargestellte Zeitfenster entspricht der eigentlichen vierwöchigen Testperiode zwischen den beiden Placebo-Kontrollperioden. Die für die Gruppe A mit dem Kombinationspräparat ermittelten Werte sind als Punkte, die für die Gruppe B mit dem Vergleichspräparat ermittelten als Striche dargestellt. Die Versuchsdurchführung erfolgte doppelblind, d.h. weder der Arzt noch der Patient wussten, welches der beiden Präparate jeweils zu Anwendung kam.

Überraschenderweise zeigen die Ergebnisse der Langzeituntersuchung, dass sich bei den Personen der Gruppe A, welche das Kombinationspräparat erhalten hatten, keine Beinträchtigung der cognitiven Leistungsfähigkeit einstellte, dass diese dagegen am Ende der vierwöchigen Beobachtungszeit sogar statistisch signifikant (Wilcoxon P<0.05) erhöht war. Bei den Personen der Vergleichgruppe B, die reines Lorazepam erhalten hatten, stellte sich hingegen, am Beobachtungsende ebenfalls statistisch signifikant, die zu erwartende negative Beeinträchtigung der cognitiven Leistungsfähigkeit ein. Eine Erhöhung der cognitiven Leistungsfähigkeit lässt sich zwar auch mit bestimmten Präparaten anderer Art, wie z.B. mit sogenannten Nootropica erreichen; im Zusammenhang mit einem Schlafmittel ist ein derartiger Effekt dagegen bisher noch nicht beobachtet worden.

Gegenstand der Erfindung ist damit eine Mischung aus Lorazepam und Diphenhydramin oder eines Säureadditionssalzes von Diphenhydramin im Gewichtsverhältnis von 1:10 - 1:50 zur Herstellung eines Schlafmittels, welches bei chronischer Einnahme sowohl als Schlafmittel wirk, als auch eine Steigerung der cognitiven Leistungsfähigkeit verursacht.

Das Schlafmittel hergestellt nach der Erfindung ist besonders geeignet für Patienten, welche unter chronischen Schlafstörungen leiden und bei denen eine weitere Beeinträchtigung ihrer bereits altersoder krankheitsbedingt reduzierten cognitiven Leistungsfähigkeit nicht in Kauf genommen werden kann oder zumindest zu vermeiden ist.

Lorazepam ist der nicht wortgeschützte Freiname (Generic name) von 7-Chlor-5-(2-chlorphenyl)-1,3-dihydro-3-hydroxy-2H-1,4-benzodiazepin-2-on. Diphenhydramin ist der Freiname für 2-(Diphenylmethoxy)-N,N-dimethylethanamin.

Als Säuren für die Bildung therapeutisch anwendbarer Säureadditionssalze von Diphenhydramin eignen sich im Prinzip alle Säuren, die gut verträgliche Salze bilden wie z.B. Salzsäure, Phosphorsäure, Salizylsäure, Acetylsalizylsäure, Weinsäure, Ascorbinsäure, 4-Sulfamidobenzosäure usw. Gewöhnlich wird das Diphenhydramin-Hydrochlorid oder die freie Base verwendet. Die andern Säureadditionssalze sind jedoch im allgemeinen ebensogut geeignet wie das Hydrochlorid. Je nachdem was für ein Salz von Diphenhydramin gewählt wird, verschiebt sich das optimale Mischungsverhältnis zwischen Lorazepam und dem Diphenhydraminsalz entsprechend dem veränderten Molekulargewicht dieses Salzes. Als besonders zweckmässig hat sich ein Gewichtsverhältnis im Bereich zwischen 1:20 und 1:35 erwiesen. Unter Verwendung des Diphenhydramin-Hydrochlorids wird ein Gewichtsverhältnis von 1:25 besonders bevorzugt.

Als orale Darreichungsformen für das Kombinationspräparat aus Lorazepam und Diphenhydramin kommen in Betracht Tabletten, Dragees, Kapseln, Pulver und Suspensionen.

Geeignete enterale Darreichungsformen sind Suppositorien oder Klistiere. Feste orale Darreichungsformen werden im allgemeinen bevorzugt. Den festen Darreichungsformen werden ausser den beiden Wirksubstanzen noch ungiftige pharmazeutische Trägersubstanzen wie Maisstärke, Weizenstärke, Talk, Natriumbicarbonat, Weinsäure, Zitronensäure, Magnesiumstearat usw. zugesetzt. Die Mischung aus den Wirkstoffen und den Zusätzen wird in der Regel zu Tabletten gepresst. Sie können nötigenfalls anschliessend noch dragiert werden, besonders wenn sie für die Pädiatrie bestimmt sind.

Für die Herstellung von Suppositorien wird die Wirkstoffmischung mit den Trägersubstanzen und Suppositorienmasse vermischt, in der Suppositorienmasse geschmolzen und zu Suppositorien vergossen oder gepresst.

Flüssige Darreichungsformen sind in der Regel wässerige Lösungen von Diphenhydramin-Salzen, in denen Lorazepam suspendiert ist. Zur Stabilisierung der Suspensionen werden Netzmittel oder Polyhole wie z.B. Polyethylenglykol oder Polypropylenglykol sowie Glykol oder Glycerinester zugesetzt.

### Beispiel 1

### Tabletten

2,5 kg Diphenhydramin-Hydrochlorid und 100 g Lorazepam werden mit Mais- oder Kartoffelstärke, 15 Milchzucker, Zellulosepulver, Magnesiumstearat und Talk zu einem Granulat verarbeitet und zu 100,000 Tabletten mit einem Gehalt von je 1 mg Lorazepam und 25 mg Diphenhydramin-Hydrochlorid gepresst.

### Beispiel 2

### Dragees

Nach Beispiel 1 hergestellte Tabletten werden noch mit einem Zuckerüberzug dragiert.

### Beispiel 3

### Tabletten

Nach Beispiel 1 hergestellte Tabletten werden mit einem Film überzogen, der aus Polymeren wie Zellulosederivaten, Polymethacrylsäureester, Polyvinylpyrrolidon, Polyethylenglykol besteht.

### Beispiel 4

### Tabletten

6,47 kg Diphenhydramin-Salizylat werden mit 100 g Lorazepam vermischt, ähnlich wie im Beispiel 1 beschrieben, granuliert und danach zu 200,000 Tabletten gepresst.

### Beispiel 5

### Kapseln

2,5 Diphenhydramin-Hydrochlorid und 0,1 kg Lorazepam werden mit den üblichen Zusätzen, wie unter 35 Beispiel 1 erwähnt, vermischt und direkt oder nach vorherigem Granulieren in 100,000 Hartgelatine-Kapseln mit einem Gehalt von je 1 mg Lorazepam und 25 mg Diphenhydramin-Hydrochlorid abgefüllt.

### Beispiel 6

### Suppositorien

2,5 kg fein pulverisiertes Diphenhydramin-Hydrochlorid und 0,1 kg fein pulverisiertes Lorazepam werden mit Suppositorienmasse, die zur Hauptsache aus hydrierten pflanzlichen Fetten besteht, vermischt und zu 100,000 Suppositorien vergossen mit einem gehalt von je 1 mg Lorazepam und 25 mg Diphenhydramin-Hydrochlorid.

## Patentansprüche

1. Verwendung einer Mischung aus Lorazepam und Diphenhydramin oder eines Säureadditionssalzes von Diphenhydramin im Gewichtsverhältnis von 1:10 - 1:50 zur Herstellung eines Arzneimittels, welches bei chronischer Einnahme gleichzeitig sowohl als Schlafmittel wirkt, als auch eine Steigerung der cognitiven Leistungsfähigkeit verursacht.

2. Verwendung einer Mischung aus Lorazepam und Diphenhydramin oder eines Säureadditionssalzes von Diphenhydramin im Gewichtsverhältnis von 1:10-1:50 zur Herstellung eines Arzneimittels, welches bei Patienten mit chronischen Schlafstörungen und alters- oder krankheitsbedingt reduzierter cognitiver Leistungsfähigkeit bei chronischer Einnahme gleichzeitig sowohl als Schlafmittel wirkt, als auch eine Steigerung der cognitiven Leistungsfähigkeit verursacht.

3. Verwendung nach Anspruch 1 oder 2, **dadurch gekennzeichnet, dass** sie Lorazepam und Diphenhydramin oder ein Säureadditionssalz von Diphenhydramin im Gewichtsverhältnis 1:20 bis 1:35, insbesondere 1:25, enthält.

## Claims

1. A use of a mixture of lorazepam and diphenhydramine or of an acid additive salt of diphenhydramine in a ratio by weight of 1:10 to 1:50 for producing a drug which, when administered chronically, simultaneously acts as a soporific and increases cognitive efficiency.

2. The use of a mixture of lorazepam and diphenhydramine or of an acid additive salt of diphenhydramine in a ratio by weight of 1:10 to 1:50 for producing a drug which, when administered chronically to patients having chronic sleep disorders and age-related or disease-induced reduced cognitive efficiency, simultaneously acts as a soporific and increases cognitive efficiency.

3. The use of claim 1 or 2, wherein the mixture contains lorazepam and diphenhydramine or an acid additive salt of diphenhydramine in a ratio by weight of 1:20 to 1:35, more specifically of 1:25.

## Revendications

1. Utilisation d'un mélange de lorazepam et de diphenhydramine ou d'un sel additionné d'acide de diphenhydramine dans une proportion de poids de 1 :10 à 1 :50 pour la fabrication d'un médicament qui, en cas de prise chronique, agit comme somnifère et provoque en même temps également une augmentation de la capacité cognitive.

2. Utilisation d'un mélange de lorazepam et de diphenhydramine ou d'un sel additionné d'acide de diphenhydramine dans une proportion de poids de 1 :10 à 1 :50 pour la fabrication d'un médicament qui, en cas de prise chronique, agit comme somnifère chez les patients présentant des perturbations chroniques du sommeil et une capacité cognitive réduite due à l'âge ou à la maladie et provoque en même temps une augmentation de la capacité cognitive.

3. Utilisation selon la revendication 1 ou 2, **caractérisée en ce qu'**elle comporte du lorazepam et de la diphenhydramine ou un sel additionné d'acide de diphenhydramine dans une proportion de poids de 1 :20 à 1 :35, en particulier 1 :25.
